# EUROPEAN PATENT APPLICATION

(11) **EP 4 745 215 A1**
(43) Date of publication of application: **20.05.2026**
(21) Application number: 24843114.0
(22) Date of filing: 16.07.2024
(51) Int. Cl.: C10L 1/14, C12N 11/00

(54) **FUEL ADDITIVE, METHOD FOR PRODUCING SAME, AND USE THEREOF**

(30) Priority: 15.07.2023 JP 2023116362
(71) Applicant: Yoshikawa, Naoya, Tokyo 152-0031 (JP); Nakamura, Hiroyasu, Yokosuka-shi, Kanagawa 238-0022 (JP)
(72) Inventor: Yoshikawa, Naoya, Tokyo 152-0031 (JP); Nakamura, Hiroyasu, Yokosuka-shi, Kanagawa 238-0022 (JP)
(74) Representative: MacLachlan IP Limited
(86) International application number: PCT/JP2024/025410
(87) International publication number: WO 2025/018324

(57) **Abstract**

To provide a fuel additive that can be applied to any organic fuel oil and can provide a fuel oil with improved combustion efficiency, and to provide a method for producing the same. A fuel additive, obtained by making an enzyme-carrying carrier by ripening an enzyme by allowing an enzyme solution, prepared by introducing a raw material enzyme containing a lipase into purified water which has been modified to sever the hydrogen bonds of the water, to stand while stirring intermittently at normal temperature, causing the enzyme to be carried by introducing a porous material and allowing the moisture to dry naturally, can improve combustion efficiency and suppress generation of greenhouse gases while suppressing the generation of harmful components in the exhaust gas without adverse effects on a combustion engine, when used as is as a fuel or when used added to a fuel.

## Description

The present invention relates to a fuel additive in which lipase is dissolved in an organic solvent, and a method for producing the same. More specifically, the present invention relates to a fuel additive capable of improving physical properties of fuel and improving combustion efficiency while suppressing a discharge amount of harmful exhaust gas components such as CO, HC, and NO_{X} PM applicable to any combustion oil and emitted by combustion, and a method for producing the same. The present invention further relates to the application of the fuel additive.

### BACKGROUND ARTS

As a heat source that heats a working fluid in a heat engine, a combustion engine that burns fuel and converts chemical energy into thermal energy has been used in various fields (for example, a so-called transportation (vehicle), a generator, or the like that carries a person or an object thereon).

An internal combustion engine in which air or the like is sucked as a working body in a combustion engine and the working fluid itself is heated by participating in a combustion reaction is a general term for various prime movers that work by burning fuel inside the engine.
These internal combustion engines include internal combustion engines based on various engines such as a reciprocating engine, a rotary engine, a gas turbine engine, a jet engine, and a rocket engine, and are widely used in various fields such as a transportation object such as an automobile, a motorcycle, a ship, and an aircraft, a generator, and the like.

In addition, the external combustion engine is a combustion engine that transmits heat generated by a separate combustion reaction or the like to the working fluid via the heat exchanger and heats the working fluid, and examples thereof include a steam engine, a Stirling engine, and a nuclear engine.

Among these internal combustion engines, petroleum-based fuel oil mainly composed of hydrocarbon such as fossil fuel, particularly heavy oil, light oil, kerosene, diesel oil, gasoline or the like is used.

Fossil fuels are depletion-type fuels and have the problem of causing environmental pollution with mining and use, and releasing carbon dioxide, which is a greenhouse gas that accelerates global warming and climate change with combustion.

From these points, attention is paid to electricity as clean energy, in particular, electricity, hydrogen, or the like as renewable energy that is clean and not depleted, as a fuel in place of fossil fuel. However, most of the existing combustion engines burn fossil fuels, all of which are substantially impossible to replace new clean fuels, in particular renewable energy. The fuel additive is generally composed of polyether amine (PEA) and is marketed as a gasoline additive, a diesel fuel additive, and an additive such as gasoline diesel fuel.

Therefore, a fuel additive represented by a gasoline additive has been proposed as a technique for modifying a fuel used in an existing combustion engine.

The gasoline additive is used by being mixed with gasoline to improve the original function of gasoline, to clean, for example, improve the fuel consumption-improving power-up engine malfunction, and the like, and further,
Removal of carbon and the like deposited on an intake/exhaust valve, a combustion chamber, an injector, or the like
Improvement of fuel economy performance by reducing friction of a cylinder and a piston ring
Prevention of rust and corrosion inside the gasoline tank
The purpose of the present invention is to smooth blow-up or the like during driving of an engine.

For example, Patent Document 1 discloses an internal combustion engine, in particular, at least one diarylamine, optionally alkylated, as a fuel oil detergent in order to clean a combustion chamber of an automobile engine (Patent Document 1, JP 2019-529604 (WO 2018/041710).

In addition, in order to prevent deterioration of fuel economy due to aging deterioration of an engine, and at the same time, to improve practical fuel consumption, and to provide a fuel additive for an internal combustion engine, which, as a fuel additive having a stable quality as one package, an additive for fuel for an internal combustion engine (octane number improver in gasoline fuel) has been proposed (Patent Document 2: Japanese Patent No. 5737730) containing polyether amine carbonate:

[R₁-COO-] [R₂-O(AO)ₘ-XH+] (1)

wherein R₁ is a chain hydrocarbon residue having 7 to 21 carbon atoms, the polyether amine moiety having a salt base is a compound represented by R₂-O (AO)ₘX (in the formula, R₂ represents a hydrocarbon residue having 8 to 50 carbon atoms, A represents an alkylene group having 2 to 6 carbon atoms, O represents oxygen, m represents an integer of 10 to 50, and X represents a hydrocarbon containing an amino group or a substituted amino group, X is (C₃H₆NH)ₙH, and n is an integer of 1 to 3.

As described above, the fuel additive improves fuel economy and the like by cleaning the inside of the engine to exhibit the original performance of the engine.

The 1990s and the petroleum supply company released a premium gas oil as a light oil having a higher added value than a general light oil (normal light oil). The premium gas oil is added with a cetane number improver (cetane number +3) and an anti-rust agent (Patent Document 3: Japanese Patent Application Laid-Open No. 5-132682 (JP 05-132682A)) which improves "cleaning agent" and self-ignition (ignition) property for removing contamination of a fuel injection system with respect to general light oil (normal light oil), and reduces white smoke at low temperature start and black smoke at high load. According to Patent Document 3, the additive used in the premium gas oil is constituted by a total of 100 parts, in which 2 to 15 parts of a low-temperature fluidity improver (ethylene-vinyl acetate copolymer-based additive), 5 to 25 parts of a cetane number-improving agent (an alkyl nitrate having 6 or 8 carbon atoms), 1 to 10 parts of a stain-preventing detergent (imide-based additive) of the fuel injection nozzle, and the balance is a solvent. Patent Document 4 (International Publication No. 2003/104360 (WO 2003/104360)) discloses a cetane number improver for diesel fuel comprising beta carotene and 2,2,4-trimethyl-6-ethoxy -1,2-dihydroquinoline in WO 2003/104360.

In addition, emulsion fuel has been proposed from the viewpoint of adding water and an additive (surfactant, emulsifier, etc.) to petroleum-based hydrocarbon oil (heavy oil, light oil, gasoline, etc.), stirring and mixing the mixture, and emulsifying the mixture, so that the amount of fuel oil used can be reduced and CO₂ is reduced accordingly. For example, Patent Document 5 (Japanese Unexamined Patent Application Publication No. 2015-124328 (JP2015-124328A)) discloses a method for producing a fuel oil, characterized in that a mixer including a cylindrical container, a center pipe extending in a central portion in the cylindrical container, and a cylindrical net disposed in a cylindrical region between an inner peripheral surface of the cylindrical container and an outer peripheral surface of the center pipe is used, and a raw material liquid containing petroleum hydrocarbon oil, water, and an additive such as a lipase-containing enzyme is spirally swirled in the cylindrical region, and the raw material liquid is passed through meshes of the cylindrical net and mixed. The 2,2,4-trimethyl-6-ethoxy -1,2-dihydroquinoline described in Patent Document 4 is a flammable brown liquid, and is incompatible with an oxidizing agent and a strong acid. Upon heating, the polymerization is performed. This compound may also polymerize upon exposure to light or air. Therefore, it lacks long-term stability.

According to Patent Document 5, since " a large number of shear turbulent flows are generated and the petroleum-based hydrocarbon oil and water can be mixed and stirred finely and efficiently, a fuel oil in which petroleum-based hydrocarbon oil and water are hardly separated can be obtained. In addition, when a lipase-containing enzyme is used as the additive, the petroleum-based hydrocarbon oil is efficiently hydrolyzed together with a large number of shear turbulence and the water to be separated is reduced. Therefore, it is possible to increase the amount of water to be incorporated in the petroleum-based hydrocarbon oil, and to obtain a fuel oil in which the petroleum-based hydrocarbon oil and water are less likely to be separated over a long period of time. The following description is given.

### PRIOR ART DOCUMENTS

### Patent Documents

Patent Document 1: JP 2019-529604 A
Patent Document 2: Japanese 5737730B
Patent Document 3: JP 05-132682A
Patent Document 4: WO 2003/104360
Patent Document 5: JP2015-124328A

### SUMMARY OF THE INVENTION

### PROBLEMS SOLVED BY THE INVENTION

As described in Patent Documents 1 to 3, the fuel additive is intended to improve fuel economy by cleaning the inside of the engine, but it mainly corresponds to an internal combustion engine used for a long period of time, and does not correspond to an engine with little deterioration or contamination.

In addition, Patent Document 4 provides a cetane number improver for fuel containing 2,2,4-trimethyl-6-ethoxy -1,2-dihydroquinoline as a main component, but when providing a gasoline modifier, 2,2,4-trimethyl-6-ethoxy -1,2-dihydroquinoline and another component are combined. Therefore, in Patent Documents 1 to 4, a single item exhibits an effect on a specific petroleum fraction, and the properties of the fuel cannot be improved by being applied to any kind from heavy oil to gasoline. Further, it is described that a plant component may be added, but the plant component is not composed only of the plant component.

Further, there is no fuel improving agent capable of increasing combustion efficiency while suppressing CO, HC, NO_{X}, PM and the like emitted by combustion.

In addition, the emulsion fuel as described in Patent Document 4 does not easily separate the water and the fuel oil, and does not guarantee that the emulsion fuel is not separated. Therefore, applications that can be applied in consideration of maintenance and the like when water and fuel oil are separated in a combustion engine are limited. In fact, there is no example in which the emulsion fuel is commercially successful.

Accordingly, it is an object of the present invention to provide a fuel additive capable of providing a fuel oil applicable to any organic combustion oil and having improved combustion efficiency, and a method for producing the same.

### MEANS FOR SOLVING PROBLEMS

As a result of intensive studies in view of such circumstances, the present inventors have found that when a lipase is added to a fuel oil as a lipase fuel additive in which a lipase is dissolved in a hydrocarbon-based oil, the combustion efficiency increases, and the discharge amount of CO and PM can be reduced while maintaining the discharge amount of NOx or the like.

That is, the first invention is a fuel additive obtained by dissolving a lipase in a hydrocarbon-based oil, characterized by being produced by the following steps:
preparing enzyme water by adding 5 g to 200 g of a raw material enzyme containing a lipase to 1 L of purified water modified by cleaving hydrogen bonds of water; allowing the resulting enzyme water to stand for 1 to 30 days while being intermittently stirred at room temperature to be aged; incorporating the enzyme water containing an aged enzyme with a porous material to cause an enzyme to be carried on the porous material, drying the porous material naturally to form an enzyme-carried carrier; adding 5 g to 500 g of the enzyme-carried carrier per 100 L of the hydrocarbon-based oil on a dry basis, and stirring the mixture while being aerated; and leaving the hydrocarbon oil into which an enzyme-carried carrier is incorporated for at least 24 hours to thereby obtain a fuel additive.
In the fuel additive of the present invention, the hydrocarbon oil is preferably gasoline, kerosene, light oil containing jet fuel, heavy oil, or biodiesel fuel. In addition, the fuel additive of the present invention is particularly preferably formed by diluting the fuel additive in an amount of 10 to 100 times the vegetable oil having a flash point of 250°C.

A second aspect of the present invention relates to a fuel oil having improved properties and exhaust gas characteristics obtained by adding 1/1000 to 1/100000 volume parts of the fuel additive to 1 vol. of the fuel oil.
In the second invention of the present invention, it is preferable that the fuel oil is a fuel oil or a mixture thereof having at least one function of increasing the cetane number, increasing the flash point, and improving fluidity in addition to the properties and the exhaust gas characteristic function thereof, and having a boiling point of 170-350°C obtained by oil conversion. In addition to the properties and the exhaust gas characteristic function, the fuel oil is preferably gasoline with an increased octane number.

A third invention is a method for producing a fuel additive obtained by dissolving a lipase in a hydrocarbon-based oil, which comprises the following steps: preparing enzyme water by adding 5 g to 200 g of a raw material enzyme containing a lipase to 1 L of purified water modified by cleaving hydrogen bonds of water; allowing the resulting enzyme water to stand for 1 to 30 days while being intermittently stirred at room temperature to be aged;
incorporating the enzyme water containing an aged enzyme with a porous material to cause an enzyme to be carried on the porous material; drying the porous material naturally to form an enzyme-carried carrier; adding 5 g to 500 g of the enzyme-carried carrier per 100 L of the hydrocarbon-based oil on a dry basis, and stirring the mixture while being aerated, and leaving the hydrocarbon oil into which an enzyme-carried carrier is incorporated for at least 24 hours to thereby obtain a fuel additive.

In a specific aspect of the present invention, the present invention relates to an enzyme-carried carrier on which an enzyme containing a lipase is carried. The carrier produced by the following steps: preparing enzyme water by adding 5 g to 200 g of a raw material enzyme containing a lipase to 1 L of purified water modified by cleaving hydrogen bonds of water; allowing the resulting enzyme water to stand for 1 to 30 days while being intermittently stirred at room temperature to be aged;
incorporating the enzyme water containing an aged enzyme with a porous material to cause an enzyme to be carried on the porous material; and drying the porous material naturally to form an enzyme-carried carrier.

In another particular aspect of the present invention, the present invention is method for reducing the emission amount of carbon dioxide discharged by combustion of a combustion engine using an organic combustion oil, the method comprising: a step of adding a fuel additive according to any one of claims 1 to 3 to the fuel oil, and a step of introducing, into the combustion engine, a fuel oil having the fuel additive being added.

### EFFECT OF THE INVENTION

When the fuel additive of the first invention containing the lipase at a high concentration in the hydrocarbon oil is used by being added to the fuel oil, the combustion efficiency is improved while suppressing the generation of harmful components in the exhaust gas without adversely affecting the combustion engine, and the generation of greenhouse gas can be suppressed. In addition, since the fuel additive of the first invention does not substantially contain moisture, there is no problem of rust at the bottom of the fuel tank due to moisture

That is, by improving the combustion efficiency, the output with respect to the unit usage amount increases. For example, when a fuel oil for a vehicle is used, fuel efficiency can be improved, and the amount of fuel used can be reduced. This reduced amount leads to a reduction in the amount of greenhouse gas generated by CO₂. In addition, for example, the amount of power generated by the fuel additive of the first invention is increased. This leads to a reduction in the amount of greenhouse gas generated by CO₂.

Further, since the lipase solution of the present invention dissolves the lipase in an organic solvent miscible with various fuel oils such as gasoline, kerosene, light oil containing jet fuel, heavy oil, or biodiesel fuel, the lipase solution can be used by being added to various fuel oils. As a result, it was possible to provide various fuel oils in which the amount of greenhouse gas generation in which lipase was dissolved was reduced.

### EMBODIMENTS FOR CARRYING OUT THE INVENTION

Hereinafter, embodiments of the present invention will be described. The terms and substances used in the present specification have the following meanings. The term "hydrocarbon-based oil" generally refers to a fossil fuel-based oil, and particularly means oil used as a fuel oil. In particular, the hydrocarbon-based oil that can be used in the present invention is oil that can be added to the fuel oil as a fuel additive. Preferred hydrocarbon oils in the present invention are light oil or kerosene, which is a petroleum fraction that is relatively easy to handle, but is not limited thereto.

The "lipase" is an enzyme that catalyzes the hydrolysis of hydrocarbons, and a lipase derived from a plant or a lipase derived from an animal is used, but in the present invention, a lipase derived from a plant such as a pineapple-derived lipase is used. Lipase is an enzyme widely distributed in not only animals but also plants, microorganisms, and the like. For example, lipase enzymes sold under the trade names such as lipase GS "Amano" 250 G, Lipase DF "Amano" 15, Lipase MER "Amano", lipase MHA "Amano" 10 SD, Lipase A "Amano" 6, Lipase R "Amano", Lipase AY "Amano" 30 SD, and the like, or lipase enzyme-containing fermented extract obtained by fermenting pineapple sold under the trade name SUPER XX from GGI World Wide Management LTD. can be used. In addition, enzyme water containing lipase as a main component can be produced by fermenting pineapple or edible mushroom. In the present invention, these enzymes are powder-like or liquid-like and collectively referred to as a raw material enzyme containing lipase. In addition to lipase, other enzymes such as bromelines may be included.

The fuel oil to be applied in the present invention is generally a fuel oil used to operate a combustion engine, and means a fuel containing hydrocarbon as a main component. Typically, among fossil fuels such as gasoline, kerosene, light oil (diesel), heavy oil and the like obtained by petroleum refining, biodiesel and the like are exemplified, but the present invention is not limited thereto. In the present invention, biodiesel fuel, which is particularly liquid fossil fuel and commonly used for many combustion engines, kerosene, light oil (diesel), heavy oil and alternative fuels thereof, is a subject. The relationship between the fuel oil, the calorific value, and the CO₂ emission coefficient is as follows.

Crude oil: unit calorific value (GI)/Id) 38.2, CO₂ emission coefficient (CO₂ conversion per heat generation amount (tCO₂)/GL): 0.0686, CO₂ conversion per unit amount (t CO₂)/t) 2.62
Gasoline: unit calorific value (GI)/Id) 34.6, CO₂ emission coefficient (CO₂ conversion per heat generation amount (tCO₂)/ GL): 0.0671, CO₂ conversion per unit amount (t CO₂) /t) 2.32
Naphtha: unit calorific value (GI)/Id) 33.6, CO₂ emission coefficient (CO₂ conversion per heat generation amount (tCO₂)/ GL): 0.0667, in terms of CO₂ per unit amount (t CO₂)/ t) 2.24
Jet fuel oil: unit calorific value (Gl)/ Id) 36.7, CO₂ emission coefficient (CO₂ conversion per heat generation amount (tCO₂)/GL): 0.0671, CO₂ conversion per unit amount (t CO₂)/t) 2.46
Kerosene: unit heat generation amount (Gl)/ Id) 36.7, CO₂ emission coefficient (CO₂ conversion per heat generation amount (tCO₂)/GL): 0.0678, CO₂ conversion per unit amount (t CO₂)/t) 2.49
Light oil: unit calorific value (GI)/Id) 37.7, CO₂ emission coefficient (CO₂ conversion per heat generation amount (tCO₂)/GL): 0.0686, CO₂ conversion per unit amount (t CO₂)/t) 2.58
A Heavy oil: Unit calorific value (GI)/Id) 39.1, CO₂ emission coefficient (CO₂ conversion per heat generation amount (tCO₂)/GL): 0.0693, CO₂ conversion per unit amount (t CO₂)/t) 2.71
B C Heavy Oil: Unit calorific value (GI)/Id) 41.9, CO₂ emission coefficient (CO₂ conversion per heat generation amount (tCO₂)/GL): 0.0715, CO₂ conversion per unit amount (t CO₂)/ t) 3.00

These fuel oils generally include paraffin-based, olefin-based, naphthenic-and aromatic-based hydrocarbons, sulfur compounds, nitrogen compounds, oxygen compounds, metal compounds, and the like, which are non-hydrocarbon components.

As described in the prior art, the "combustion engine" in the present invention is a combustion engine that burns a fuel oil and converts chemical energy into thermal energy as a heat source that heats a working fluid in a heat engine. In particular, various engines, such as reciprocating engines, rotary engines, gas turbine engines, jet engines, rocket engines, etc. that work by combusting the fuel oil inside the engine include internal combustion engines based on various engines such as reciprocating engines, rotary engines, gas turbine engines, jet engines, rocket engines, and the like (vehicles); an external combustion engine such as a boiler that is a combustion engine that transmits heat generated by a combustion reaction separate from an internal combustion engine such as a vehicle or a generator to a working fluid via a heat exchanger and heats the working fluid.

The term "combustion efficiency" is defined as the ratio of the amount of heat actually generated in the combustion process to the low heat generation amount of the fuel supplied to the combustion engine, and this value is 1 when the combustion is ideally advanced (complete combustion), but this value is not 1 because there is a loss due to incomplete combustion. The incomplete combustion loss occurs because the fuel is discharged to the outside of the combustion device without completely burning, and due to the inclusion of the incomplete combustion component in the exhaust gas, the unburned fuel component remains in the residual ash content. The improvement in combustion efficiency is required to eliminate the cause of these incomplete combustion losses, and in the present invention, the combustion efficiency by fuel is improved. In other words, the combustion efficiency is a ratio that becomes power (output) among combustion in the combustion engine, and the incomplete combustion loss is energy discarded as it is without power.

In addition, in the present invention, fuel efficiency may be shown as a measure of combustion efficiency. For example, fuel economy is used as a scale for measuring combustion efficiency in a vehicle or the like. In this case, the fuel efficiency is an index indicating a travel distance per unit capacity of fuel (gasoline, light oil, or the like) or a certain distance.

Greenhouse gas is a gas that is in the atmosphere and produces greenhouse effect by absorbing a portion of infrared radiation emitted from the ground surface, and water vapor, carbon dioxide, methane, dinitrogen monoxide, chlorofluorocarbon, and the like correspond to greenhouse gases. The main greenhouse gas increased by human activity includes carbon dioxide, methane, dinitrogen monoxide, and chlorofluorocarbon gas, and particularly carbon dioxide generated due to a large amount of fossil fuel consumption including the fuel oil of the present invention is a problem, and the reduction of the carbon dioxide is required.

The improvement in combustion efficiency is to increase the output (combustion amount) per unit consumption (e.g. 1 liter) of the fuel, and the increase in combustion efficiency will reduce the amount of fuel consumption and reduce the amount of carbon dioxide emissions. Therefore, the combustion oil having improved combustion efficiency in the present invention has the same meaning as the fuel oil having a reduced greenhouse gas generation amount.

### (Production Method)

A fuel additive obtained by dissolving a lipase in a hydrocarbon-based oil according to the present invention is obtained by: adding 5 g to 200 g of a raw material enzyme containing a lipase to purified water 1 L to prepare enzyme water; leaving the prepared enzyme water for 1 to 30 days while intermittently stirring the prepared enzyme water for 1 to 30 days to mature the enzyme; charging the enzyme water containing the aged enzyme into a porous material to carry the enzyme on the porous material; naturally drying the moisture to form an enzyme-carried carrier; charging 10 g to 1000 g of the enzyme-carried carrier into a hydrocarbon-based oil on a dry basis, stirring the enzyme-carried carrier while blasting, and leaving the hydrocarbon oil into which the enzyme-carried carrier is charged for at least 24 hours. Such a fuel additive is dissolved in a hydrocarbon-based oil in which an enzyme mainly composed of lipase is an organic solvent at a high concentration.

By dissolving the lipase in the hydrocarbon-based oil by such a predetermined method, a fuel additive containing a high-concentration lipase can be obtained.

In the present invention, it is preferable to use refined water obtained by treating such a raw material with purified water, particularly pure water of a cluster, for example, pure water of a milli-Q level, and a water purifier sold as a pi water purifier for business use from an Alike Emum Co. Ltd. To such purified water 1 L, 5 g to 200 g of a raw material enzyme containing the lipase described above is charged and stirred to prepare enzyme water. The pi water is water having enhanced permeability and water absorbency by passing through such a water purifier to cut a hydrogen bond. Similarly, it is preferable to use water having enhanced permeability and water absorbency obtained by cleaving a hydrogen bond by applying magnetic force energy to water or passing the water through a special ceramic. Cleaving the hydrogen bond of water in this manner is referred to as modification of water in the present invention. The modified water is referred to as modified water.

When the raw material enzyme containing lipase is less than 5 g per 1 L of purified water, a high-concentration lipase solution cannot be prepared, and even if it exceeds 200 g, lipase cannot be dissolved, resulting in waste.

The prepared enzyme water is then stirred at room temperature and left to stand for 1 week to 30 days, preferably 1 week to 2 weeks. After the enzyme water aged in this way is filtered to remove impurities, a porous material, for example, activated carbon, carbon powder, or zeolite carrier is charged. The porous material at this time is preferably one having a particle size of about 0.1 to 5 mm, and is used in such an amount that the porous material is immersed in enzyme water.

After being left in this way, the porous material in which the enzyme is immersed in natural drying, for example, a basket, a mesh plate, or the like is transferred, and dried or preferably dried on the sun. When dried in this manner, a carrier in which a considerable amount of enzyme is carried in the porous material is obtained (intermediate).

The intermediate is charged with a hydrocarbon-based oil, for example, 5 g to 500 g per 100 L of kerosene, stirred while being aerated, and left to stand for at least 24 hours, preferably 36 hours or more, more preferably 60 hours or more. By processing in this manner, the fuel additive of the present invention can be produced. It should be noted that this intermediate can be used, for example, for the production of a fuel additive of the present invention after another production and transportation. Accordingly, the present invention is also within the scope of rights.

Since the fuel additive is an oil solution (referred to as a stock solution) in which a lipase is dissolved at a high concentration in a hydrocarbon-based oil that can be mixed with a fuel oil, the fuel additive can be mixed with the fuel oil at a high dilution rate.

The properties of the fuel oil can be improved by adding the fuel additive of the present invention to, for example, 1/1000 to 1/100000 volumes of the fuel additive with respect to the volume of the fuel oil 1. The addition amount of the fuel additive of the present invention can be appropriately changed depending on the concentration of the lipase in the fuel additive, and the optimum amount is determined by, for example, the amount of the raw material enzyme containing the silkworm added to the purified water and the amount of the intermediate body added to the hydrocarbon-based oil serving as the solvent.

In the case of a fuel additive for a fuel for a consumer such as a fuel oil for a vehicle or a fuel oil for a small-sized generator, the fuel additive can be further diluted with a hydrocarbon-based oil to facilitate handling even with a small amount of fuel oil.

Furthermore, in a preferred embodiment of the present invention, a fuel additive obtained by diluting the stock solution of the fuel additive of the present invention with an oil having a flash point of 250°C of 10 to 100 times, preferably a vegetable oil, and more preferably a palm oil, cottonseed oil, rapeseed oil, Western brassicum oil or the like can be used as the fuel additive. With this configuration, the fuel additive of the present invention can be distributed as a product that is not a hazardous material having a flash point of 250°C or less.

When the fuel additive of the present invention containing the lipase at a high concentration in the hydrocarbon oil is used by being added to the fuel oil, the combustion efficiency is improved while suppressing the generation of harmful components in the exhaust gas without adversely affecting the combustion engine, and the generation of greenhouse gas can be suppressed. In addition, since the fuel additive of the present invention does not substantially contain moisture, there is no problem of rust at the bottom of the fuel tank due to moisture.

That is, by improving the combustion efficiency, the output with respect to the unit usage amount increases. For example, when a fuel oil for a vehicle is used, fuel efficiency can be improved, and the amount of fuel used can be reduced. This reduced amount leads to a reduction in the amount of greenhouse gas generated by CO₂. In addition, for example, the amount of power generated by the fuel additive of the first invention is increased. This leads to a reduction in the amount of greenhouse gas generated by CO₂.

In a preferred embodiment of the present invention, the fuel additive of the present invention is used by dissolving lipase as a concentrated liquid in kerosene or light oil, and adding the concentrated liquid to light oil, kerosene, or other fuel oil. In this case, the fuel additive of the present invention can be rephrased as a fuel oil having a reduced greenhouse gas generation amount.

As described above, the lipase solution is preferably dissolved in kerosene or light oil as a concentrated liquid, and when the concentrated liquid is added to light oil, kerosene, or other fuel oil, the lipase permeates the entire fuel, and it is preferable that the lipase solution is allowed to stand for several hours, for example, 1 to 24 hours until the fuel itself is modified.

By allowing the mixture to stand for a predetermined time in this manner, the aromatic component is olefinated by the action of the lipase in the fuel oil, and the elements such as N and S in the heterocycle are removed via the aqueous phase. In a preferred embodiment of the present invention, the fuel additive of the present invention can be diluted by a predetermined dilution amount, preferably 10 to 100 times, with oil having low flammability exceeding 250° C, preferably vegetable oil, such as palm oil, rapeseed oil, and Western brassicum. By diluting with oil having a low flammability exceeding 250°C as described above, the fuel additive of the present invention can be handled as a product having low flammability.

As described above, when the fuel additive of the present invention is used as a fuel or in addition to the fuel, the combustion efficiency is improved while suppressing the generation of harmful components in the exhaust gas without adversely affecting the combustion engine, and the generation of greenhouse gas can be suppressed.

That is, by improving the combustion efficiency, the output with respect to the unit usage amount increases. For example, when a fuel for a vehicle is used, fuel efficiency can be improved, and the amount of fuel used can be reduced. This reduced amount leads to a reduction in the amount of greenhouse gas generated by CO₂. In addition, for example, in a case where the fuel additive of the present invention is added to a predetermined fuel and used as a fuel of a generator, the combustion efficiency is improved, and the power generation amount with respect to the unit usage amount of the fuel is increased. This leads to a reduction in the amount of greenhouse gas generated by CO₂. That is, regardless of the type of the fuel oil, the fuel oil to which the lipase solution of the present invention is added can provide a fuel capable of burning at a high combustion efficiency while discharging a relatively clean exhaust gas. High combustion efficiency means that high power and high fuel consumption can be achieved using the same fuel. That is, the use amount of the same fuel can be improved from 10% to 20%. This can reduce CO₂ for the same fuel (see Example 4).

Further, since the lipase solution of the present invention dissolves the lipase in an organic solvent miscible with various fuel oils such as gasoline, kerosene, light oil containing jet fuel, heavy oil, or biodiesel fuel, the lipase solution can be used by being added to various fuel oils. As a result, it was possible to provide various fuel oils in which the amount of greenhouse gas generation in which lipase was dissolved was reduced.

In addition, the fuel additive of the present invention can increase combustion efficiency while suppressing CO, HC, NO_{X} PM, and the like emitted by combustion which cannot be imagined by the prior art.

Although the embodiment of the present invention has been described above, the present invention is not limited to these embodiments. For example, by adding the lipase solution of the present invention to the fuel of the target combustion engine and leaving it for a predetermined time, it is possible to suppress the carbon dioxide emission amount of the combustion engine. Therefore, the present invention is extended to a carbon dioxide emission amount reduction method for reducing the emission amount of carbon dioxide discharged by combustion of a combustion engine using an organic fuel oil including a step of adding the lipase solution of the present invention to the organic fuel oil to be used and a step of introducing the organic fuel to which the lipase solution is added to the combustion engine.

### EXAMPLE

### Example 1: Production of Fuel Additive

Enzyme water was prepared by adding 30 g of SUPER XX from GGI World Wide Management LTD as a raw material enzyme containing lipase in 10 L of purified water treated by a pi water purifier for business use from an Aecum Co. Ltd.

The enzyme water was appropriately stirred and left at room temperature (20 to 28° C.) for 1 week.

After the impurities were filtered, 300 g of zeolite (particle size: 0.1 mm) was placed and left overnight, the treated zeolite was transferred to a 100-mesh steel plate and dried on the sun, and a carrier on which a lipase enzyme was carried was prepared.

The fuel additive of the present invention was obtained by putting a carrier in which lipase enzyme is carried on 100L of kerosene and aerating under stirring for 3 days.

### (Example 2)

The fuel additive manufactured in Example 1 was left to stand for 24 hours in addition to 1 cc per 100 L of diesel oil to prepare the diesel fuel oil 1 of the present invention.

Table 1 shows the number of rotations, the concentration of exhaust gas, the output, the fuel consumption, and the like when the diesel oil of the present invention and the non-added diesel oil are added (an output test by 4.65 liters of the detrite diesel 4 cylinder). As shown in Table 1, it can be seen that the addition of the lipase solution of the present invention improves the output and fuel economy while dramatically reducing the emissions of carbon monoxide (CO) and soot (C).

**[Table 1]**

| | Operating Conditions | High Speed Diesel Oil | High Speed Diesel Oil+ Fuel Additive |
|---|---|---|---|
| 1 Rotation No (RPM) | Dead Slow Speed | 923 | 924 |
| | Slow Speed | 1101 | 1102 |
| | Half | 1235 | 1223 |
| | Full | 1340 | 1330 |
| 2 HC Concentration (PPM) | Dead Slow Speed | 1625 | 1733 |
| | Slow Speed | 1660 | 1698 |
| | Half | 1675 | 1687 |
| | Full | 1700 | 1695 |
| 3 CO% | Dead Slow Speed | 8.50 | 8.58 |
| | Slow Speed | 8.95 | 7.66 |
| | Half | 9.25 | 7.35 |
| | Full | 9.40 | 7.25 |
| 4 CO₂% | Dead Slow Speed | 14.43 | 10.36 |
| | Slow Speed | 13.60 | 9.79 |
| | Half | 12.70 | 9.60 |
| | Full | 12.70 | 9.40 |
| 5 O₂% | Dead Slow Speed | 0.93 | 3.60 |
| | Slow Speed | 1.10 | 4.50 |
| | Half | 1.00 | 5.70 |
| | Full | 0.83 | 7.30 |
| 6 C % | Dead Slow Speed | 3.90 | 3.60 |
| | Slow Speed | 5.30 | 4.50 |
| | Half | 6.30 | 5.70 |
| | Full | 9.00 | 7.30 |
| 7 Output (KW) | Dead Slow Speed | 31.70 | 33.20 |
| | Slow Speed | 44.60 | 46.34 |
| | Half | 57.60 | 63.04 |
| | Full | 65.90 | 69.45 |
| 8 Fuel Ratio KG/Hr | Dead Slow Speed | 9.14 | 7.32 |
| | Slow Speed | 11.50 | 10.32 |
| | Half | 14.50 | 12.22 |
| | Full | 16.70 | 13.46 |

### (Example 3)

In addition, 1 cc of the fuel additive of Example 1 was added per 1 L of the diesel fuel oil, and the running test was performed using a diesel vehicle (truck: VOLVO FH 12). As a result, the fuel efficiency in the case of traveling with a normal diesel engine from the travel distance 325, 193 miles to 425, and 857 miles was 39.26 liters/100 km, and when the fuel additive of the present invention was added, 35.62 liter/100 km was achieved, and 9.3% fuel cost and carbon dioxide were reduced.

### (Example 4)

As in Example 3, when the fuel additive of Example 1 was added to gasoline, the combustion efficiency (fuel efficiency) of the gasoline vehicle was improved from 10% to 20%.

In the present invention, for example, in a case where the fuel efficiency of a gasoline vehicle having a fuel consumption of 10 km/L is improved to 12 km/L when the fuel additive of the present invention is added to gasoline, the distance capable of traveling with a fuel of 100 L is 1000 km, and the emission amount of CO2 is 232.166 kg-CO2. On the other hand, the fuel required for traveling 100 km with the fuel when the lipase solution of the present invention is added is 83.33L, and the discharge coefficient thereof is 193.464 kg-CO2.

### (Example 5)

As in Examples 3 and 4, it was found that by adding the lipase solution of the present invention to the heavy oil used for the ship, it is possible to reduce the fuel of 8 to 11%.

### (Example 6)

A fuel additive was produced in the same manner as in Example 1, except that 10L of kerosene was used instead of 100L of kerosene L in Example 1. 90L of a palm oil was added to the fuel additive to prepare a fuel additive of Example 6.

Similar effects were confirmed as a result of the same experiment as in Example 3 and Example 4 for the fuel additive of Example 6. As described above, the product can be used as a product having low flammability.

### INDUSTRIAL AVAILABILITY

When the fuel additive of the first invention containing the lipase at a high concentration in the hydrocarbon oil is used by being added to the fuel oil, the combustion efficiency is improved while suppressing the generation of harmful components in the exhaust gas without adversely affecting the combustion engine, and the generation of greenhouse gas can be suppressed. In addition, since the fuel additive of the first invention does not substantially contain moisture, there is no problem of rust at the bottom of the fuel tank due to moisture

That is, by improving the combustion efficiency, the output with respect to the unit usage amount increases. For example, when a fuel oil for a vehicle is used, fuel efficiency can be improved, and the amount of fuel used can be reduced. This reduced amount leads to a reduction in the amount of greenhouse gas generated by CO₂. In addition, for example, the amount of power generated by the fuel additive of the first invention is increased. This leads to a reduction in the amount of greenhouse gas generated by CO₂.

Further, since the lipase solution of the present invention dissolves the lipase in an organic solvent miscible with various fuel oils such as gasoline, kerosene, light oil containing jet fuel, heavy oil, or biodiesel fuel, the lipase solution can be used by being added to various fuel oils. As a result, it was possible to provide various fuel oils in which the amount of greenhouse gas generation in which lipase was dissolved was reduced.

## Claims

1. A fuel additive obtained by dissolving a lipase in a hydrocarbon-based oil, produced by the following steps:
preparing enzyme water by adding 5 g to 200 g of a raw material enzyme containing a lipase to 1 L of purified water modified by cleaving hydrogen bonds of water;
allowing the resulting enzyme water to stand for 1 to 30 days while being intermittently stirred at room temperature to be aged;
incorporating the enzyme water containing an aged enzyme with a porous material to cause an enzyme to be carried on the porous material,
drying the porous material naturally to form an enzyme-carried carrier,
adding 5 g to 500 g of the enzyme-carried carrier per 100 L of the hydrocarbon-based oil on a dry basis, and stirring the mixture while being aerated, and
leaving the hydrocarbon oil into which an enzyme-carried carrier is incorporated for at least 24 hours to thereby obtain a fuel additive.

2. The fuel additive according to claim 1, wherein the hydrocarbon oil is gas oil, heavy oil, or biodiesel fuel containing gasoline, kerosene, and jet fuel.

3. A fuel additive obtained by diluting the fuel additive according to claim 1 or 2 with a vegetable oil having a flash point of more than 250°C in an amount of 10 to 100 times.

4. The fuel additive according to any one of claims 1 to 3, which has improved properties and exhaust gas characteristics obtained by adding 1/1000 to 1/100000 volume parts of the fuel additive to 1 capacity part of the fuel oil.

5. The fuel oil according to claim 3, wherein the fuel oil is a fuel oil or a mixture thereof having at least one function of increasing the cetane number, increasing the flash point, and improving fluidity in addition to the properties and the exhaust gas characteristic function thereof, and having a boiling point of 170-350°C obtained by oil conversion.

6. The fuel oil of claim 3, wherein the fuel oil is gasoline with an increased octane number in addition to its properties and exhaust gas characteristic functions.

7. A method for producing a fuel additive obtained by dissolving a lipase in a hydrocarbon-based oil, which comprises the following steps:
preparing enzyme water by adding 5 g to 200 g of a raw material enzyme containing a lipase to 1 L of purified water modified by cleaving hydrogen bonds of water;
allowing the resulting enzyme water to stand for 1 to 30 days while being intermittently stirred at room temperature to be aged;
incorporating the enzyme water containing an aged enzyme with a porous material to cause an enzyme to be carried on the porous material,
drying the porous material naturally to form an enzyme-carried carrier,
adding 5 g to 500 g of the enzyme-carried carrier per 100 L of the hydrocarbon-based oil on a dry basis, and stirring the mixture while being aerated, and
leaving the hydrocarbon oil into which an enzyme-carried carrier is incorporated for at least 24 hours to thereby obtain a fuel additive.

8. An enzyme-carried carrier on which an enzyme containing a lipase is carried, produced by the following steps:
preparing enzyme water by adding 5 g to 200 g of a raw material enzyme containing a lipase to 1 L of purified water modified by cleaving hydrogen bonds of water;
allowing the resulting enzyme water to stand for 1 to 30 days while being intermittently stirred at room temperature to be aged;
incorporating the enzyme water containing an aged enzyme with a porous material to cause an enzyme to be carried on the porous material, and
drying the porous material naturally to form an enzyme-carried carrier.

9. A method for reducing the emission amount of carbon dioxide discharged by combustion of a combustion engine using an organic combustion oil, the method comprising
a step of adding a fuel additive according to any one of claims 1 to 3 to the fuel oil,
and a step of introducing, into the combustion engine, a fuel oil having the fuel additive being added.
